# EUROPEAN PATENT SPECIFICATION

(11) **EP 2 183 596 B1**
(45) Date of publication and mention of the grant of the patent: **03.10.2012**
(21) Application number: 08779222.2
(22) Date of filing: 07.07.2008
(51) Int. Cl.: G01N 33/564, G01N 33/543, C07K 16/28

(54) **METHOD AND MEANS FOR PREDICTION OF SYSTEMIC LUPUS ERYTHEMATOSUS SUSCEPTIBILITY**
VERFAHREN UND MITTEL ZUR VORHERSAGE DER ANFÄLLIGKEIT FÜR SYSTEMISCHEN LUPUS ERYTHEMATODES
PROCEDE ET MOYEN DE PREDICTION D'UNE SENSIBILITE SYSTEMIQUE AU LUPUS ERYTHEMATOSUS

(30) Priority: 24.07.2007 SE 0701778
(43) Date of publication of application: 12.05.2010
(73) Proprietor: ITH Immune Therapy Holdings AB, 171 76 Stockholm (SE)
(72) Inventor: WINQVIST, Ola, S-75653 Uppsala (SE); KARLSSON, Mikael C. I., S-17770 Järfälla (SE)
(74) Representative: White, Nina Louise
(86) International application number: PCT/SE2008/000433
(87) International publication number: WO 2009/014476

(56) References cited:
- WO-A1-99/42831
- WO-A1-2006/050573
- WO-A2-2004/080385
- YAMADA Y ET AL: "Scavenger receptor family proteins: roles for atherosclerosis, host defense and disorders of the central nervous system", CMLS CELLULAR AND MOLECULAR LIFE SCIENCES, BIRKHAUSER VERLAG, HEIDELBERG, DE, vol. 54, no. 7, 1 July 1998 (1998-07-01), pages 628-640, XP002959151, ISSN: 1420-682X, DOI: DOI:10.1007/S000180050191
- ARREDOUANI M S ET AL: "The structure and function of MARCO, a macrophage class a scavenger receptor", CELLULAR AND MOLECULAR BIOLOGY (NOISY-LE-GRAND), vol. 50, no. Suppl. S, 2004, pages 657-665, XP8134492, ISSN: 0145-5680
- WERMELING FREDRIK ET AL: "Class A scavenger receptors regulate tolerance against apoptotic cells, and autoantibodies against these receptors are predictive of systemic lupus.", THE JOURNAL OF EXPERIMENTAL MEDICINE 1 OCT 2007 LNKD- PUBMED:17893199, vol. 204, no. 10, 1 October 2007 (2007-10-01), pages 2259-2265, XPxp8134441, ISSN: 0022-1007
- MUNOZ L.E. ET AL.: 'SLE-a disease of clearance deficiency?' RHEUMATOLOGY vol. 44, 2005, pages 1101 - 1107, XP003023850
- SHOENFELD Y. ET AL.: 'Autoantibodies against Protective Molecules - C1q, C-Reactive Protein, Serum Amyloid P, Mannose-Binding Lectin, and Apolipoprotein A1' ANNALS OF THE NEW YORK ACADEMY OF SCIENCES vol. 1108, 2007, pages 227 - 239, XP003023851

## Description

### FIELD OF THE INVENTION

The present invention relates to a method and a means for predicting the risk of a person developing systemic Lupus erythematosus.

### BACKGROUND OF THE INVENTION

Apoptotic cells are considered to be a major source for autoantigens in autoimmune diseases such as systemic Lupus erythematosus (SLE). In agreement with this, defective clearance of apoptotic cells has been shown to increase disease susceptibility. Still, little is known about how apoptotic cell derived self antigens activate autoreactive B cells and where this takes place.

A specific B cell subtype in the marginal zone of the spleen is thought to be the source of auto-antibodies in several models of autoimmunity (1,2). These so-called marginal zone B cells (MZB), essential for defence and responses against blood-borne bacteria, are phenotypically characterized by high IgM and complement receptor expression (3). As an example of MZBs involvement in self reactivity, B cells are rescued from deletion in the MZB population in mice expressing a B cell receptor with affinity for self antigens (4). Autoreactive MZBs can also be activated spontaneously without T cell help and the role of these B cells as producers of autoantibodies is supported by several studies (5,6).

The source of auto-antigens for B cell activation in systemic Lupus erythematosus (SLE) is thought to be apoptotic cells and defects in apoptotic cell clearance increase susceptibility to SLE (7.8).

WO 2004/080385 provides a method of reducing an inflammatory response in a subject. The method comprises providing to a subject in need thereof a therapeutically effective amount of an agent capable of reducing activity and/or expression of a scavenger receptor or of an effector thereof, thereby reducing the inflammatory response in the subject.

### OBJECTS OF THE INVENTION

It is an object of the invention to provide a method of predicting the risk of a person developing systemic Lupus erythematosus susceptibility.

It is another object of the invention to provide a means for use in the method.

Further objects of the invention will become obvious from the following summary of the invention, a number of preferred embodiments illustrated in a drawing, and the appended claims.

### SUMMARY OF THE INVENTION

The present invention is based on the insight that apoptotic cells are taken up by specific scavenger receptors expressed on macrophages in the splenic marginal zone and that persons deficient in these receptors have a lower threshold of autoantibody response. Most important, autoantibodies against scavenger receptors are found in serum before the onset of clinical symptoms in SLE-prone mice and in diagnosed SLE patients. Without wishing to be bound by theory it is believed that autoantibodies towards scavenger receptors can alter the response to apoptotic cells, affect tolerance and thus promote disease progression. The autoantibodies of the invention lower tolerance to nuclear antigens, opening up for subsequent B cell activation by apoptotic cells, giving antibody responses such as anti-DNA that ultimately lead to disease.

Since the autoantibodies of the invention can be detected before disease onset they have predictive value as early indicators of SLE.

According to the present invention is disclosed a method of predicting the risk of a person developing systemic lupus susceptibility comprising the detection of autoantibodies to class A scavenger receptors. In a preferred embodiment the method comprises the detection of autoantibodies towards MARCO and/or SR-A.

In particular, the method of the invention comprises providing a first reagent antibody against autoantibody to class A. scavenger receptors, preferably anti-MARCO antibody and/or anti-SR-A antibody, contacting a sample of serum from a person to be tested for susceptibility to SLE with the reagent antibody, determining a complex formed by the reagent autoantibody to class A scavenger receptors, in particular with ANTI-MARCO antibody with autoantibody to class scavenger receptors, in particular with anti-MARCO antibody and/or anti-SR-A antibody. Optionally, providing a reagent antibody of the invention comprises raising said antibody.

According to a preferred aspect of the invention the method comprises providing a support coated with autoantibody to class A scavenger receptor, in particular soluble MARCO or soluble SR-A, adding serum from a person to be tested for susceptibility to SLE to the support followed by incubation, washing the support, contacting the washed support with a secondary antibody capable of forming a complex with antibody from the serum bound to MARCO or SR-A on the support followed by incubation, detecting the complex thus formed. It is preferred for the detection of the complex to include quantification.

According to a preferred aspect of the invention is disclosed a kit comprising a support coated with human anti-MARCO or human anti-SR-A antibodies, and a secondary antibody capable of forming a complex with antibody from human serum capable of binding to MARCO or SR-A.

The invention will now be explained in more detail by reference to preferred embodiments illustrated in a drawing.

### SHORT DESCRIPTION OF THE FIGURES

Figs. 1 - 6 are confocal laser-scanning microscope images showing that apoptotic cells bind MARCO and SR-A and that apoptotic cells are trapped in the marginal zone of the spleen;
Figs. 7 - 11 are diagrams, and Fig. 12 is a confocal laser-scanning microscope image showing that Class A scavenger receptors regulate tolerance against intravenously injected apoptotic cells;
Figs. 13 and 14 are diagrams showing the absence of an apoptotic cell clearance defect in scavenger receptor deficient mice;
Figs. 15 - 17 are diagrams, and Fig. 18 is a confocal laser-scanning microscope image showing the presence of anti-MARCO antibodies in SLE prone mice;
Figs. 20 and 21 are diagrams showing IgG anti-MARCO and IgG anti-DNA reactivity in sera from SLE patients.

### DESCRIPTION OF PREFERRED EMBODIMENTS

### Methods

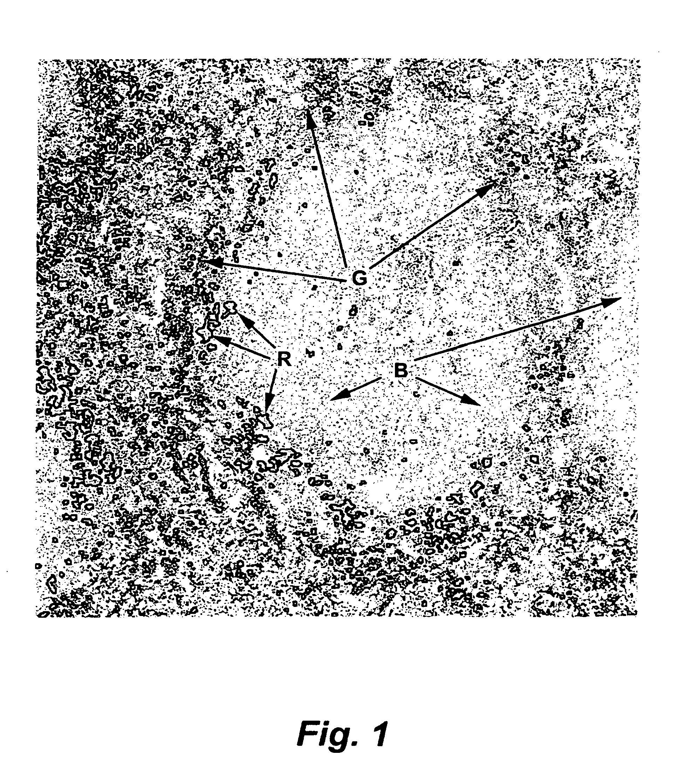

Mice. Mice were age and sex matched, kept and bred under pathogen-free conditions according to local ethical guidelines. SR-A-/-, MARCO-/- and double-knockout mice (DKO) (15, 16) were backcrossed to the C57BL/6 strain for >10 generations. (NZB x NZW) F1 mice were purchased from The Jackson Laboratory. In most studies, wild-type mice were of the C57BL/6 strain. In the experiments illustrated in Fig. 1 BALB/c mice were used, because the anti-mouse SR-A mAb 2F8 does not recognize the receptor in the C57BL/6 strain. Mice were maintained at the MBB animal facility and the work was approved by the local ethical committee.

Apoptosis induction and injections. Syngeneic thymocytes were prepared with 40 µm cell strainer (Becton Dickinson) and washed twice in sterile PBS. The cells were cultured for 6 h in RPMI 1640 supplemented with 10% bovine serum, 2 mM glutamine, 100 IU/ml penicillin, 100 µg/ml streptomycin (Gibco) and 1 µM dexamethasone (Sigma) in 6-well plates (3 mL/well) at a concentration of approximately 107 cells/mL. The cells were harvested and thoroughly washed three times with sterile PBS. The apoptotic phenotype was evaluated with annex in V-FITC and propidium iodine staining (Becton Dickinson) in FACSCalibur flow cytometer and Cellquest software (Becton Dickinson). About 85% of the injected cells were annex in V+. Age and sex matched (10-week-old females) wild type (wt; C57BL/6), SR-A-/-, MARCO-/- and double knockout (DKO) mice (n=8 per genotype) were immunized weekly for four weeks with 10⁷ apoptotic cells in sterile PBS i.v. in the tail vein (17). Serum samples were collected weekly, from the tail artery, starting two days before the first injection.

Immunohistochemistry and anti-DNA responses. Syngeneic thymocytes were prepared and stained with 2 µM PKH26 (Sigma) as described by the manufacturer before induction of apoptosis. Cells (6×10⁷) were injected i.v. into BALB/c mice (n=4). Spleens were collected at 45 min and 5 h later, and were frozen in OCT medium (Sakuru). Six-µm thin sections were cut in a cryostat microtome. After overnight drying the slides were fixed in ice cold acetone for 5 min and stored at -75°C. Before staining slides were blocked with 5% goat serum (Dako) and 4% BSA in PBS. The antibodies used were: rat anti-MARCO27, rat anti-SR-A unlabelled and biotinylated (Serotec), anti-B220-bio (Becton Dickinson), anti-CD11c-FITC (Becton Dickinson), anti-rat Alexa488 (Invitrogen) and streptavidin-Qdot605 (Invitrogen). Images were collected using a confocal laser-scanning microscope (TCS SP2; Leica Microsystems) equipped with one argon and two HeNe lasers. Anti-dsDNA autoantibodies were measured as previously described (28). Briefly, ELISA plates were precoated with methylated BSA and then coated with calf thymus DNA (Sigma). After blocking, serum samples were added. Anti-dsDNA reactivity was measured with alkaline phosphate-conjugated anti-mouse IgG, IgM, IgG1, IgG2a, IgG2b and IgG3 antibodies (Southern Biotechnology). All samples were run in duplicates and corrected for background binding. Hep2000 slides (Immuno concept) were used for ANA assay as described by the manufacturer.

Binding assays. CHO cells were transfected with murine SR-A, MARCO, or a control vector as described (29). Apoptotic cells were added in a ratio of 5:1 or 10:1 to transfected cells in DMEM/10 mM Hepes, pH 7.5. After 1 h incubation at 37°C, the cells were washed five times with PBS, and then processed as described (29). The cells were stained for MARCO and SR-A, then incubated with Alexa 488-conjugated secondary antibody and DAPI (Invitrogen). Binding was detected with Leica DMRB microscope coupled to Retiga Exi Cooled camera.

### Autoantibody response against anti-scavenger receptors.

Soluble MARCO was purified as previously described (30). MaxiSorp 96 well plates (Nunc) were coated with 1-2 µg/mL sMARCO in PBS overnight at 4°C. Plates were washed 5 times with PBS + 0.05% Tween 20 and blocked with an excess of blocking buffer for 2 h in at room temperature (RT). Blocking buffer was tapped off and serum samples were added diluted in blocking buffer followed by 2 h incubation at RT. The plates were then washed as above and secondary antibodies were added; anti-human IgG-HRP (DAKO) or anti-mouse IgG-AP (Southern Biotechnology). After 1 h incubation at RT, plates were washed and substrate was added. All samples were run in duplicate and corrected for background binding.

Statistical analysis. Non-parametric Mann-Whitney U test was performed using Statistica software (StatSoft Inc). p<0.05 was considered significant.

Clearance evaluation in KO mice. Two approaches were used to evaluate if the knock out mice had deficiencies in clearing of apoptotic cells. First, 10-week-old female wild type and KO mice (n=6 per genotype) were bled without prior treatment from the tail artery into tubes containing heparin (Leo Pharma), which were kept on ice. Erythrocytes were lysed by two rounds of ACK treatment. The cells were stained with annex in V-FITC and analysed by flow cytometry. Second, syngeneic thymocytes were labelled with 0.1 µM CFSE (Molecular Probes) as described by the manufacturer before induction of apoptosis as described above. Cells (10⁸) were injected i.v. in age, sex and weight matched wild type and KO mice (n=6-8 per genotype). Blood was collected from the tail vein after 30 min and 3 h. After lysis of erythrocytes, the CFSE+ population was analysed by flow cytometry.

### EXAMPLE I

*Localization of apoptopic cells to the marginal zone of the spleen.* Activation/selection of auto-reactive MZBs, a possible source of antigen in innate B cell activation, needs to include access to autoantigen. For this reason the localization of apoptotic cells to the marginal zone of the spleen was investigated.

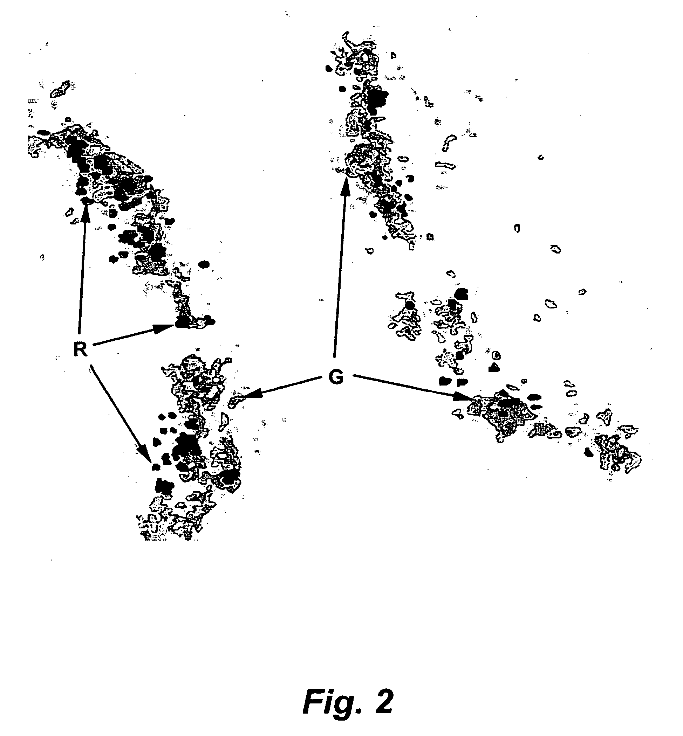

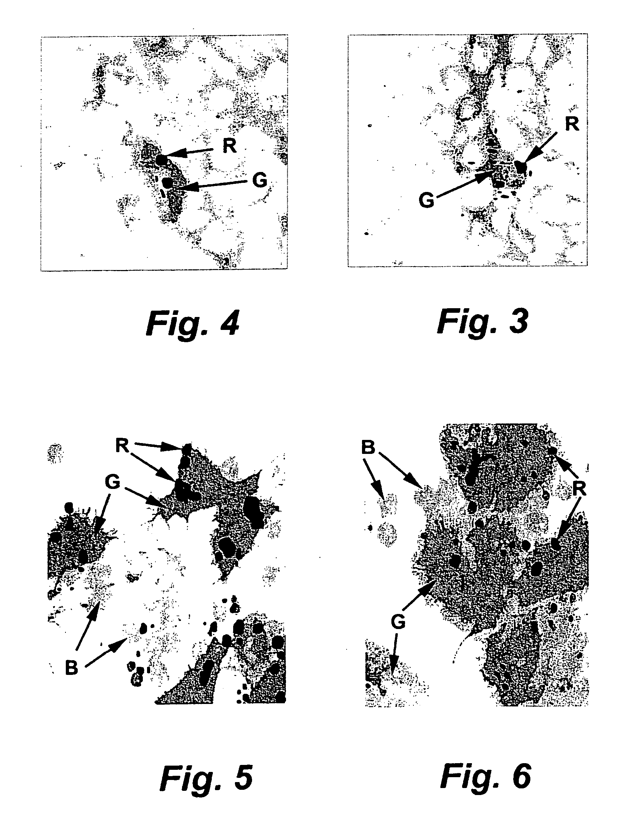

Wild type (wt) mice were injected i.v. with B 220 labelled apoptotic cells B220. Spleens were collected at different time points. The injected apoptotic cells were trapped by phagocytes in the marginal zone of the spleen, 30 min after injection (Fig. 1). At 5 h from injection, fewer labelled apoptopic cells were found in the marginal zone of the spleen, indicating swift clearance (data not shown). Several subtypes of potent APCs reside in the marginal zone, including dendritic cells (DC), known to be able to ingest apoptotic cells (9,10). However, even though some apoptotic cells were taken up by CD11c+ DCs, it was found that, at the early time points of 30 min and 5 h, the apoptotic material primarily bound to marginal zone macrophages (MZMO) (Fig. 2). These macrophages reside in close contact with MZBs and can be distinguished by their expression of specific scavenger receptors called MARCO (Fig. 3) and SR-A (Fig. 4) (2,11). SR-A and MARCO belong to the class A scavenger receptor family which binds an array of self and foreign ligands including oxidated-LDL and bacterial antigens (2,12). SR-A is known to bind apoptotic cells (13), as confirmed in an experiment with SR-A transfected CHO cells (Fig. 6). In a corresponding experiment was shown that MARCO shares this ability (Fig. 5). In this assay clustering of apoptotic cells could not be seen on non-transfected cells stained with DAPI (not shown).

These findings indicate that MZMOs regulates the response and access to self antigens in the marginal zone for recognition by MZBs and DCs. The inability of specific macrophages to ingest apoptotic cells in the germinal centre leads to auto-antibody production. In an analogous system, in contrast, proper clearance of apoptotic cells by macrophages has been shown to be important in a microenvironment where B cells are activated/selected (14).

### EXAMPLE 2

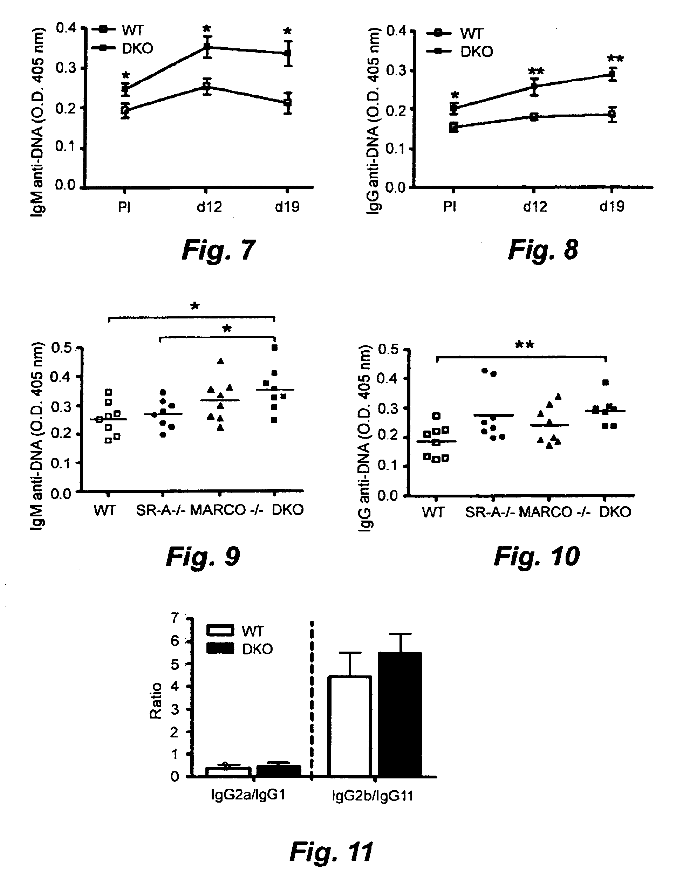

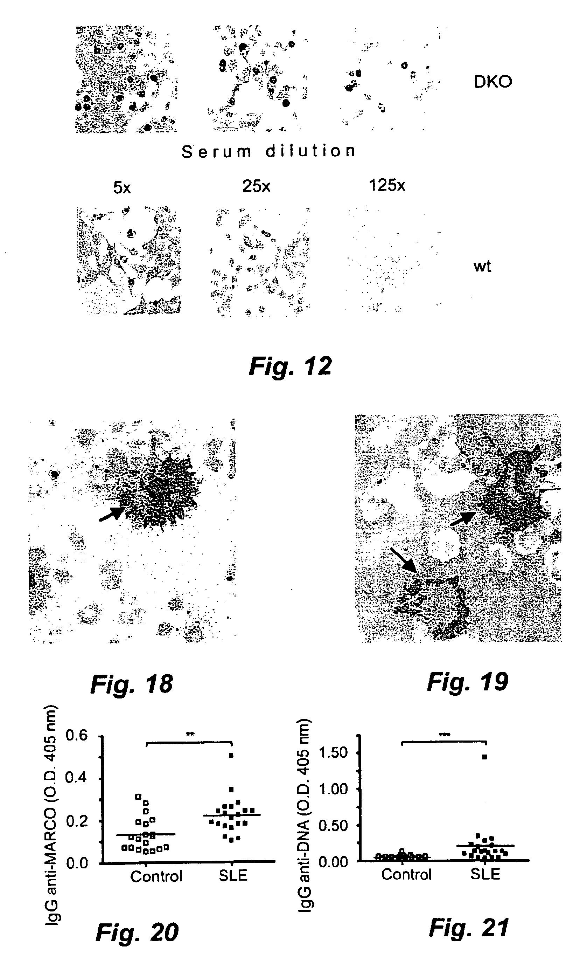

*Regulation of self response by MARCO and SR-A positive macrophages.* To explore whether MARCO and SR-A positive macrophages are involved in regulating self responses the ability to maintain tolerance after injection of syngeneic apoptotic cells, without adjuvant, was investigated in mice deficient in one or both of these receptors (15,16). Apoptotic cells were injected weekly four times in wild type, SR-A-/-, MARCO-/- and double knockout mice (DKO), in a protocol adopted from Mevorach et al. (17), and anti-DNA responses were measured with ELISA. All receptor-deficient mice had an elevated and more rapid response to apoptotic cells compared to control mice and the phenotypes were additive, resulting in the highest response in the DKO mice. The DKO mice also displayed significantly higher levels of IgM anti-DNA (Fig. 7) and IgG anti-DNA (Fig. 8) without provocation by apoptotic cells suggesting spontaneous development of anti-DNA autoimmune responses. No major differences could be seen with regard to Ig isotype of anti-DNA antibodies in the different knockout mice, except that MARCO deficiency seems to contribute more to IgM anti-DNA titres and SR-A deficiency to IgG anti-DNA titres (Figs. 9 and 10). The specific IgG response was mainly of the IgG2b subclass in all mice and the DKO mice tended to have a higher IgG2a/b:IgG1 ratio than wt, which is suggestive of a higher degree of pathogenicity (18) (Fig. 11). In agreement with the anti-DNA ELISA data, anti-nuclear autoantibodies (ANA) in injected DKO mice showed a homogeneous nuclear staining pattern at day 26 from injection, which was present at a higher titration than in the wild type mice, indicating DNA as a major autoantigen (Fig. 12).

### EXAMPLE 3

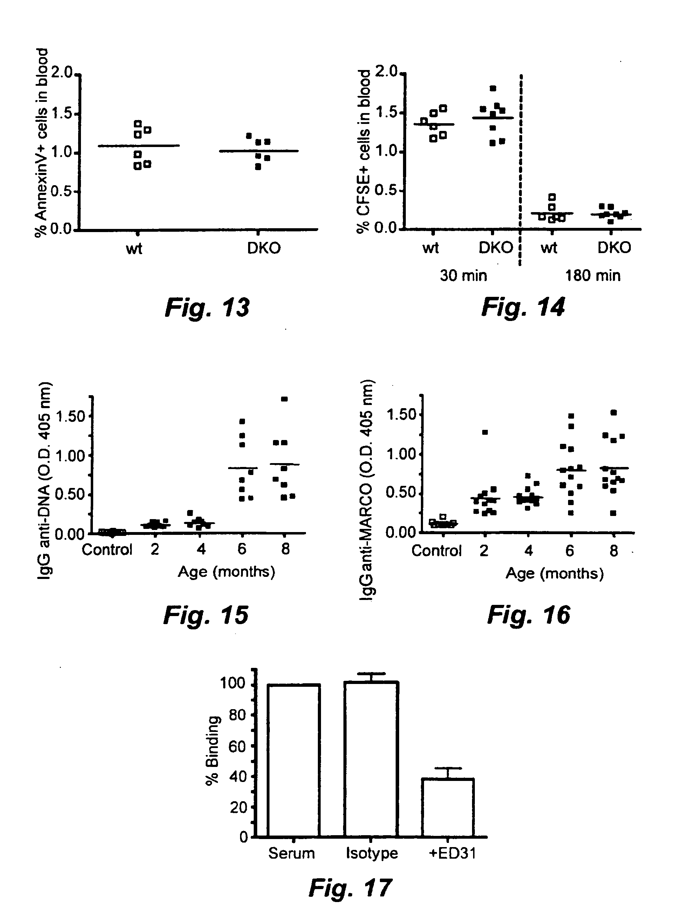

*Deletion of receptors leads to decrease apoptopic clearance.* An explanation for the increased anti-DNA response is that deletion of the receptors leads to decreased clearance of apoptotic cells, in turn resulting in increased self antigen load. With this in mind, it was investigated whether the mice displayed any defects in the clearance of apoptotic cells. The number of circulating apoptotic cells in the blood did not differ between DKO mice and wild type mice, and there were no detectable differences in clearance of i.v. injected apoptotic cells (Fig. 13).

The MARCO gene is located on chromosome 1 (122 Mb from the centromer), nearby but slightly proximal to the major lupus susceptibility loci of the NZB, NZW and BXSB mice (19). Nevertheless, one of the BXSB loci, Bxs2, linked to ANA and anti-DNA production peaks at the D1Mit12 marker (122 Mb) in the vicinity of Marco (20) suggesting that Marco might contribute to SLE susceptibility. The SR-A gene, on chromosome 8 in mice, has not been shown to reside within the known susceptibility loci for SLE or other autoimmune diseases. Even though Marco is found within a susceptibility locus for anti-DNA responses, these receptors might also cause autoimmunity by acting as autoantigens. In such an alternative mechanism, blocking autoantibodies could potentially interfere with efficient uptake of apoptotic cells, thereby promoting anti-DNA autoimmune responses. One example is the autoantibodies towards the structurally related complement protein C1q that are found in SLE. In this case, however, the C1q autoantibodies apparently increase the severity of glomerulonephritis, rather than affecting clearance of apoptotic cells (21).

### EXAMPLE 4

*Detection of autoantibodies to class A scavenger receptors.* To investigate a possible development of autoantibodies binding class A scavenger receptors, sera from lupus-prone (NZB x NZW) F1 mice were tested for IgG anti-MARCO activity by ELISA (22). Sera from 2, 4, 6 and 8 months old mice were tested, spanning the development of disease that starts at 5 months as determined by IgG anti-DNA levels (Fig. 15). Significant levels of IgG anti-MARCO antibodies were detected as early as after 2 months and peaked at 6 months (Fig. 16). The binding of the autoimmune sera to MARCO could be blocked by adding an antibody towards the ligand binding domain (Fig. 17). The presence of anti-MARCO autoantibodies in (NZB x NZW) F1 mice was further confirmed by staining transfected cells expressing either MARCO (Fig. 18) or SR-A protein (Fig. 19), implicating class A scavenger receptors as autoantigens in SLE.

### EXAMPLE 5

Patients. Twenty SLE patients diagnosed with SLE at a young age classified by ACR criteria and 19 matched healthy individuals were selected. Sera from young SLE patients with low anti-DNA titres were chosen since mice mouse data indicate that the anti-MARCO reactivity can be found early in development of the disease. The work was approved by the local ethical committee. Class A scavenger receptors are highly conserved among species and MARCO has a 74% amino acid identity between mice and man (24). The SLE patients showed significantly higher reactivity towards MARCO protein (Fig. 20) than towards anti-DNA protein (Fig. 21).

### References

1. Lopes-Carvalho, T. et al., Development and selection of marginal zone B cells. Immunol Rev 197, 192-205 (2004).
2. Kraal, G. et al., New insights into the cell biology of the marginal zone of the spleen. Int Rev Cytol 250, 175-215 (2006).
3. Martin, F. et al., Marginal-zone B cells. Nat Rev Immunol 2, 323-35 (2002).
4. Li, Y. et al., Anti-DNA B cells in MRL/1pr mice show altered differentiation and editing pattern. J Exp Med 196, 1543-52 (2002).
5. Enzler, T. et al. Alternative and Classical NF-kappaB Signaling Retain Autoreactive B Cells in the Splenic Marginal Zone and Result in Lupus-like Disease. Immunity 25, 403-15 (2006).
6. Mandik-Nayak, L. et al., Autoreactive marginal zone B cells are spontaneously activated but lymph node B cells require T cell help. J Exp Med 203, 1985-98 (2006).
7. Kim, S.J. et al., Opsonization of apoptotic cells and its effect on macrophage and T cell immune responses. Ann N Y Acad Sci 987, 68-78 (2003).
8. Casciola-Rosen, L.A. et al., Autoantigens targeted in systemic lupus erythematosus are clustered in two populations of surface structures on apoptotic keratinocytes. J Exp Med 179, 1317-30 (1994).
9. Morelli, A.E. et al., Internalization of circulating apoptotic cells by splenic marginal zone dendritic cells: dependence on complement receptors and effect on cytokine production. Blood 101, 611-20 (2003).
10. Steinman, R.M. et al., The induction of tolerance by dendritic cells that have captured apoptotic cells. J Exp Med 191, 411-6 (2000).
11. Peiser, L. et al., The function of scavenger receptors expressed by macrophages and their role in the regulation of inflammation. Microbes Infect 3, 149-59 (2001).
12. Elomaa, O. et al. Cloning of a novel bacteria-binding receptor structurally related to scavenger receptors and expressed in a subset of macrophages. Cell 80, 603-9 (1995).
13. Platt, N. et al., Role for the class A macrophage sca venger receptor in the phagocytosis of apoptotic thymocytes in vitro. Proc Natl Acad Sci U S A 93, 12456-60 (1996).
14. Hanayama, R. et al., Autoimmune disease and impaired uptake of apoptotic cells in MFG-E8-deficient mice. Science 304, 1147-50 (2004).
15. Suzuki, H. et al., A role for macrophage scavenger receptors in atherosclerosis and susceptibility to infection. Nature 386, 292-6 (1997).
16. Chen, Y. et al., Defective microarchitecture of the spleen marginal zone and impaired response to a thymus-independent type 2 antigen in mice lacking scavenger receptors MARCO and SR-A. J Immunol 175, 8173-80 (2005).
17. Mevorach, D. et al., Systemic exposure to irradiated apoptotic cells induces autoantibody production. J Exp Med 188, 387-92 (1998).
18. Nimmerjahn, F. et al., Divergent immunoglobulin g subclass activity through selective Fc receptor binding. Science 310, 1510-2 (2005).
19. Kono, D.H. et al., Genetics of SLE in mice. Springer Semin Immunopathol 28, 83-96 (2006).
20. Hogarth, M.B. et al., Multiple lupus susceptibility loci map to chromosome 1 in BXSB mice. J Immunol 161, 2753-61 (1998).
21. Trouw, L.A. et al., Anti-C1q autoantibodies deposit in glomeruli but are only pathogenic in combination with glomerular C1q-containing immune complexes. J Clin Invest 114, 679-88 (2004).
22. Burnet, F.M. et al., The natural history of the NZB/NZW F1 hybrid mouse: a laboratory model of systemic lupus erythematosus. Australas Ann Med 14, 185-91 (1965).
23. Karlsson, M.C. et al., Macrophages control the retention and trafficking of B lymphocytes in the splenic marginal zone. J Exp Med 198, 333-40 (2003).
24. Elomaa, O. et al., Structure of the human macrophage MARCO receptor and characterization of its bacteria-binding region. J Biol Chem 273, 4530-8 (1998).
25. Weller, S. et al., Human b lood IgM "memory" B cells are circulating splenic marginal zone B cells harboring a prediversified immunoglobulin repertoire. Blood 104, 3647-54 (2004).
26. Maksimowicz-McKinnon, K. et al., Predictors of Carotid Atherosclerosis in Systemic Lupus Erythematosus. J Rheumatol (2006).
27. van der Laan, L.J. et al., Macrophage scavenger receptor MARCO: in vitro and in vivo regulation and involvement in the anti-bacterial host defense. Immunol Lett 57, 203-8 (1997).
28. McGaha, T.L. et al., Restoration of tolerance in lupus by targeted inhibitory receptor expression. Science 307, 590-3 (2005).
29. Brannstrom, A. et al., Arginine residues in domain V have a central role for bacteria-binding activity of macrophage scavenger receptor MARCO. Biochem Biophys Res Commun 290, 1462-9 (2002).
30. Sankala, M. et al., Characterization of recombinant soluble macrophage scavenger receptor MARCO. J Biol Chem 277, 33378-85 (2002).

### Legends to Figures

For rendering in the figures colour images were inverted in Adobe® Photoshop. Red (R) zones are marked by hand, and sample areas indicated by arrows. Sample green (G) and blue (B) areas are also indicated by arrows.
*Figure 1**.* PKH26 labelled (red) apoptotic cells were injected i.v. in BALB/c mice. Spleens collected after 30 min were stained with anti-CD11c (DCs, green) and anti-B220 (B cells, pseudo-coloured blue); Leica confocal system.
*Figure 2*. A corresponding serial spleen cryostat section stained with anti-MARCO (marginal zone macrophages, green); Leica confocal system.
*Figures 3 and 4*. A corresponding serial spleen cryostat section of marginal zone macrophage binding apoptotic cells (red), stained with both anti-MARCO (green; *Fig. 3*) and anti-SR-A (green, *Fig. 4*), at higher magnification; Leica confocal system.
*Figures 5 and 6**.* In vitro binding assay using CHO cells transfected with murine MARCO (*Fig. 5*) or SR-A (*Fig. 6*) and then incubated with labelled apoptotic cells (red). Cells stained with anti-MARCO or anti-SR-A (green), respectively, and with DAPI nuclear staining (blue); Leica DMRB microscope.
*Figure 7*. 10⁷ Syngeneic apoptotic cells were injected i.v. four times weekly in wild type and DKO mice (C57BL/6 background). IgM anti-DNA response in serum were measured pre-immune (PI), at day 12 and day 19. Data are shown as mean ± standard deviation (n=8 per genotype).
*Figure 8*. 10⁷ Syngeneic apoptotic cells were injected i.v. four times weekly as above). IgG anti-DNA response in serum was measured pre-immune (PI), at day 12 and day 19 (n=8 per genotype).
*Figures 9 and 10*. 10⁷ Syngeneic apoptotic cells were injected i.v. four times weekly in wild type and in MARCO^{-/-}, SR-A^{-/-}, and DKO mice (C57BL/6 background). The anti-DNA response in serum at day 12 (IgM, Fig. 9) and at day 19 (IgG, Fig. 10) was measured.
*Figure 11*. Subclass analysis of the anti-DNA response at day 26, after the fourth injection in a week of apoptotic cells in wild type and DKO mice. Data are shown as mean ± standard deviation of the O.D. 405 nm ratio between IgG2a/IgG2b and IgG1, (n=8 per genotype).
*Figure 12*. Representative anti-nuclear antigen (ANA) pattern from DKO and wt mice after the fourth injection (d26) in a week of apoptotic cells in wild type and DKO mice. * = p<0.05, ** = p<0.01 (non-parametric Mann-Whitney U-test).
*Figure 13*. The amount of circulating apoptotic cells in the blood of wild type and DKO mice measured with annexinV-FITC in a Ca2+ rich buffer. The samples were kept on ice during all steps of the experiment to reduce the risk of de novo apoptosis. Data showing % annexinV+ cells in the total cell population.
*Figure 14**.* Syngeneic apoptotic cells (10⁸) were labelled with CFSE and injected i.v. in wild type and DKO mice; 30 and 180 min after the injection, blood was collected and CFSE+ cells were counted with flow cytometry. Data show % CFSE+ cells in the total cell population.
*Figure 15*. IgG anti-DNA levels in 2, 4, 6 and 8 months old (NZB x NZW) F1 mice. Controls are 2.5 months old C57BL/6 mice.
*Figure 16*. IgG anti-MARCO reactivity in 2, 4, 6 and 8 months old (NZB x NZW) F1 mice and 2.5 months old C57BL/6 mice.
*Figure 17*. Binding to MARCO in the anti-MARCO ELISA blocked with a monoclonal antibody (ED31) against MARCO, but not with an isotype control (n=2).
*Figures 18 and* 19. CHO cells transfected with murine MARCO (Fig. 18) or SR-A (Fig. 19) stained with sera from (NZB x NZW) F1 mice and anti-mouse IgG-FITC. Arrows indicate transfected cells stained by the mouse sera.
*Figure 20**.* sMARCO or blocking buffer coated ELISA plates were incubated with sera from SLE patients (n=20) and healthy individuals (n=19). Data shown as anti-MARCO data minus anti-block buffer data, to reduce the level of binding to the block buffer.
*Figure 21*. IgG anti-DNA activity in SLE patients and healthy individuals measured by ELISA. By linear regression analysis it was shown that the data from the experiments in Fig. 20 and 21 did not correlate.

## Claims

1. A method of predicting the risk of a person developing systemic Lupus erythematosus susceptibility comprising the detection of autoantibody to class A scavenger receptors.

2. The method of claim 1, wherein the autoantibody is MARCO and/or SR-A autoantibody.

3. A method of predicting the risk of a person developing systemic Lupus erythematosus susceptibility, comprising providing a first reagent antibody against autoantibody to class A scavenger receptors; contacting a sample of serum from a person to be tested for SLE susceptibility with said first reagent antibody; and determining a first complex formed by said first reagent antibody with a serum sample component.

4. The method of claim 3, wherein providing a first reagent antibody of the invention comprises raising said antibody.

5. The method of claim 3 or 4, wherein said first reagent antibody is selected from anti-MARCO antibody and anti-SR-A antibody.

6. The method of any of claims 3 to 5, comprising providing a support coated with said first reagent antibody; contacting the support with serum from a person to be tested for SLE susceptibility; incubating the serum in contact with the support for a period of time sufficient to form said first complex bound to the support; washing the support; providing a second reagent antibody capable of forming a second complex with said serum component bound to the support; contacting the washed support with said second reagent antibody; incubating the support in contact with said second reagent antibody for a time sufficient to form said second complex; detecting said second complex.

7. The method of claim 6, comprising quantification of said second complex.

8. The method of claim 5 or 6, wherein said second reagent antibody is selected from anti-human IgG, in particular anti-human IgG-HRP, and anti-mouse IgG, in particular anti-mouse IgG-AP.

## Patentansprüche

1. Verfahren zur Vorhersage des Risikos einer Person, eine Anfälligkeit für systemischen Lupus erythematodes zu entwickeln, umfassend den Nachweis eines Autoantikörpers gegen Klasse A Scavenger-Rezeptoren,

2. Verfahren nach Anspruch 1, wobei der Autoantikörper der MARCO- und/oder SR-A-Autoantikörper ist.

3. Verfahren zur Vorhersage des Risikos einer Person, eine Anfälligkeit für systemischen Lupus erythematodes zu entwickeln, umfassend das Bereitstellen eines ersten Antikörper-Reagenzes gegen einen Autoantikörper gegen Klasse A Scavenger-Rezeptoren; das Inkontaktbringen einer Serumprobe einer Person, die auf SLE-Anfälligkeit getestet werden soll, mit dem ersten Antikörper-Reagenz; und das Bestimmen eines ersten von dem ersten Antikörper-Reagenz mit einem Bestandteil der Serumprobe gebildeten Komplexes.

4. Verfahren nach Anspruch 3, wobei das Bereitstellen eines ersten Antikörper-Reagenzes der Erfindung das Züchten des Antikörpers umfasst.

5. Verfahren nach Anspruch 3 oder 4, wobei das erste Antikörper-Reagenz aus einem Anti-MARCO-Antikörper und einem Anti-SR-A-Antikörper ausgewählt ist.

6. Verfahren nach einem der Ansprüche 3 bis 5, umfassend das Bereitstellen eines Trägers, der mit dem ersten Antikörper-Reagenz beschichtet ist; das Inkontaktbringen des Trägers mit dem Serum einer Person, die auf SLE-Anfälligkeit getestet werden soll; das Inkubieren des Serums in Kontakt mit dem Träger für eine Zeitspanne, die ausreichend ist, um den ersten an den Träger gebundenen Komplex zu bilden; das Waschen des Trägers; das Bereitstellen eines zweiten Antikörper-Reagenzes, das einen zweiten Komplex mit dem an den Träger gebundenen Serumbestandteil bilden kann; das Inkontaktbringen des gewaschenen Trägers mit dem zweiten Antikörper-Reagenz; das Inkubieren des Trägers in Kontakt mit dem zweiten Antikörper-Reagenz für eine Zeitspanne, die ausreichend ist, um den zweiten Komplex zu bilden; das Nachweisen des zweiten Komplexes.

7. Verfahren nach Anspruch 6, umfassend das Quantifizieren des zweiten Komplexes.

8. Verfahren nach Anspruch 5 oder 6, wobei das zweite Antikörper-Reagenz aus Anti-humanem IgG, insbesondere Anti-humanem IgG-HRP, und Anti-Maus IgG, insbesondere Anti-Maus IgG-AP ausgewählt ist.

## Revendications

1. Procédé permettant de prévoir le risque que présente une personne de développer une sensibilité au lupus érythémateux disséminé comprenant la détection d'un auto-anticorps dirigé contre les récepteurs de désactivation de classe A.

2. Procédé de la revendication 1, dans lequel l'auto-anticorps est un auto-anticorps MARCO et/ou SR-A.

3. Procédé permettant de prévoir le risque que présente une personne de développer une sensibilité au lupus érythémateux disséminé, comprenant le fait de fournir un premier anticorps réactif contre un auto-anticorps dirigé contre les récepteurs de désactivation de classe A ; de mettre en contact un échantillon sérique d'une personne à tester pour la sensibilité SLE avec ledit premier anticorps réactif ; et de déterminer un premier complexe formé par ledit premier anticorps réactif avec un composant d'échantillon sérique.

4. Procédé de la revendication 3, dans lequel le fait de fournir un premier anticorps réactif de l'invention comprend le fait de provoquer la production dudit anticorps.

5. Procédé de la revendication 3 ou 4, dans lequel ledit premier anticorps réactif est choisi parmi un anticorps anti-MARCO et un anticorps anti-SR-A.

6. Procédé de l'une quelconque des revendications 3 à 5, comprenant le fait de fournir un support revêtu avec ledit premier anticorps réactif ; de mettre en contact le support avec le sérum d'une personne à tester pour la sensibilité SLE ; d'incuber le sérum en contact avec le support pendant une durée suffisante pour former ledit premier complexe lié au support ; de laver le support ; de fournir un deuxième anticorps réactif capable de former un deuxième complexe avec ledit composant sérique lié au support ; de mettre en contact le support lavé avec ledit deuxième anticorps réactif ; d'incuber le support en contact avec ledit deuxième anticorps réactif pendant une durée suffisante pour former ledit deuxième complexe ; de détecter ledit deuxième complexe.

7. Procédé de la revendication 6, comprenant la quantification dudit deuxième complexe.

8. Procédé de la revendication 5 ou 6, dans lequel ledit deuxième anticorps réactif est choisi parmi l'anti-IgG humaine, notamment l'anti-IgG-HRP humaine, et l'anti-IgG de souris, notamment l'anti-IgG-AP de souris.
